(19) Europäisches Patentamt
European Patent Office
Office européen des brevets

(11) Veröffentlichungsnummer: **0 074 072**
**B2**

(12) # NEUE EUROPÄISCHE PATENTSCHRIFT

(45) Veröffentlichungstag der neuen Patentschrift: **12.04.89**

(51) Int. Cl.[4]: **C 07 C 91/26**, C 07 C 89/02

(21) Anmeldenummer: **82108022.3**

(22) Anmeldetag: **01.09.82**

(54) **Verfahren zur kontinuierlichen Herstellung von Salzen des Cholins.**

(30) Priorität: **09.09.81 DE 3135671**

(43) Veröffentlichungstag der Anmeldung:
**16.03.83 Patentblatt 83/11**

(45) Bekanntmachung des Hinweises auf die Patenterteilung:
**02.05.85 Patentblatt 85/18**

(45) Bekanntmachung des Hinweises auf die Entscheidung über den Einspruch:
**12.04.89 Patentblatt 89/15**

(84) Benannte Vertragsstaaten:
**BE CH DE FR GB LI**

(56) Entgegenhaltungen:
**DD-A- 99 569**
**DE-B- 1 518 419**
**DE-B- 2 115 094**
**DE-C- 655 892**
**JP-A-52 014 708**

(73) Patentinhaber: **BASF Aktiengesellschaft, Carl-Bosch-Strasse 38, D-6700 Ludwigshafen (DE)**

(72) Erfinder: **Distler, Harry, Dr., In den Hahndornen 5, D-6719 Bobenheim (DE)**
Erfinder: **Schneider, Rolf, Dr., Feldbergstrasse 21, D-6800 Mannheim (DE)**
Erfinder: **Wulz, Klaus, Dr., Gerolfstrasse 9, D-6715 Lambsheim (DE)**

EP 0 074 072 B2

## Beschreibung

Die Erfindung betrifft ein Verfahren zur kontinuierlichen Herstellung von Salzen des Cholins durch Vermischung von Trimethylamin, Ethylenoxid und einer Säure und Umsetzung in Gegenwart von Wasser bei einem pH oberhalb 9 bis 12 und einer Temperatur von 50 bis 80 °C.

Es ist aus Houben-Weyl, «Methoden der Organischen Chemie», Bd. 11/2, S. 610, bekannt, dass man tertiäre Amine mit Ethylenoxid in Gegenwart von Wasser zu Cholin umsetzen kann. Weitere Varianten der Synthese beschreibt die DD-PS Nr. 99 569. Einige Verfahren stellen die Salze, im allgemeinen Cholinchlorid, her, z.B. durch Umsetzung von Trimethylammoniumchlorid mit Ethylenoxid oder von Ethylenoxid, Trimethylamin und Salzsäure. Im allgemeinen wird bei erhöhter Temperatur und im alkalischen pH-Bereich gearbeitet.

Die Hauptreaktion läuft nach folgendem Schema ab:

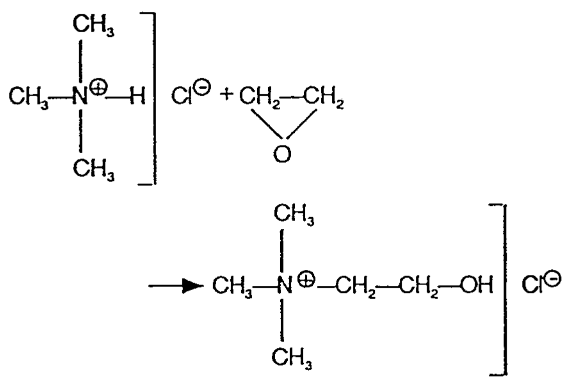

Als Nebenreaktion tritt häufig Chlorhydrin auf, das sich durch Reaktion von HCl und Ethylenoxid bildet. Ein Gehalt von Chlorhydrin macht das Cholinsalz für die meisten Anwendungen ungeeignet. Daneben wird fast immer Etherbildung beobachtet, die wegen der Toxizität des Chlorhydrins unterdrückt werden muss. Glykol und Polyglykole wurden gleichfalls bei der Synthese von Cholinsalzen als Nebenprodukte beobachtet. Schliesslich zeigt das Cholin selbst im stark alkalischen Bereich eine deutliche Zersetzungstendenz, was äusserlich durch Gelbfärbung der Reaktionslösung Cholinchlorid bei längerem Stehen zu erkennen ist. Die Wärmeentwicklung der Reaktion ist so stark, dass Nebenproduktbildung (z.B. Glykolbildung) begünstigt wird. Schon Spuren von Verunreinigungen in der Salzsäure oder im Trimethylamin können Nebenreaktionen auslösen. Mineralsäuren beschleunigen die Bildung von Glykol, das Trocknungsprobleme liefert. Trimethylaminüberschuss stört durch seinen Fischgeruch die Weiterverarbeitung des Cholins. Überschüssiges Trimethylammoniumchlorid kann die Verwendung des Cholins als Futtermittel erheblich behindern. Die Zersetzungstendenz der Cholinbase steigt mit zunehmender Konzentration und Temperatur. Die Synthesen waren bisher fast ausschliesslich diskontinuierlich. DD-PS Nr. 99 569 beschreibt auch eine kontinuierliche Umsetzung:

1. Stufe: Trimethylamin und Salzsäure zu Trimethylammoniumchlorid;

2. Stufe: Tetramethylammoniumchlorid und Ethylenoxid zu Cholinchlorid. Die Ausgangsstoffe müssen sehr rein sein, und Reaktionszeiten bis zu 120 min werden benötigt.

Um vorgenannte Schwierigkeiten zu verringern, schlägt die DD-PS ein weiteres Kontinueverfahren vor, bei dem in einer 1. Stufe aus Ethylenoxid und einem grossen Überschuss wässerigem Trimethylamin zunächst Cholinbase hergestellt wird, dann in einer 2. Stufe vorzugsweise eine teilweise Neutralisation der Cholinbase mit Salzsäure vorgenommen wird, in einer 3. Stufe das noch in der Lösung vorhandene überschüssige Trimethylamin mit einem Überschuss Ethylenoxid praktisch restlos zur Cholinbase umgesetzt wird, die im Anschluss daran sofort (Stufe 4) mit Salzsäure neutralisiert wird. In allen 4 Stufen sind eine Reihe von Parametern zu beobachten, z.B. Temperatur, Druck, Verweilzeit, Konzentrationsverhältnis, pH.

Alle diese Verfahren sind im Hinblick auf einfachen und wirtschaftlichen Betrieb und teilweise auf Ausbeute und Reinheit unbefriedigend.

Es ist bereits aus der GB-PS Nr. 1 060 256 bekannt, dass bei dem kontinuierlichen Verfahren zur Herstellung von Cholinchlorid aus Trimethylammoniumchlorid und Ethylenoxid eine wesentliche Schwierigkeit in der leichten Bildung von Nebenprodukten (Braunfärbung) besteht. Solche Nebenstoffe sind teilweise giftig bzw. besitzen unangenehmen Geruch oder Geschmack, und das so verunreinigte Cholin ist für die Herstellung von Viehfutter ungeeignet (S. 2, Zeilen 2 bis 16). Es wird ausdrücklich darauf hingewiesen, dass nur das in der Patentschrift beschriebene Verfahren ein Cholinchlorid, das gleichzeitig keine hohe Verfärbung und keinen hohen Gehalt an Ethylenchlorhydrin und an hochmolekularen Polyoxyethylen zeigt, liefert (S. 2, Zeilen 22 bis 32). Bei diesem Verfahren wird das Cholinsalz in einer ersten Stufe hergestellt, isoliert und dann in einer zweiten Stufe zunächst mit Ethylenoxid bei Temperaturen zwischen Raumtemperatur und 120 °C und einem Druck von zwischen 5 und 20 bar während 30 bis 120 min und dann in einer dritten Stufe bei mindestens 60 °C unter demselben Überdruck innerhalb von höchstens 40 min umgesetzt. Die Reaktoren der Stufen 2 und 3 sollen keine Gasphase enthalten. Für die kontinuierliche Herstellung von Cholinchlorid werden nur 2- oder 3stufige Verfahren, nämlich a) Umsetzung von Trimethylamin mit Ethylenoxid zu Cholin; dann Umsetzung von Cholin mit Salzsäure zu Cholinchlorid; oder b) Umsetzung von Trimethylaminchlorid mit Ethylenoxid, was eine vorherige Stufe der Herstellung des Trimethylaminsalzes voraussetzt (S. 1, Zeilen 13 bis 18) beschrieben. Als vorteilhaftes Verfahren wird das beanspruchte 3stufige Verfahren (Anspruch) hervorgehoben.

DE-B Nr. 2 115 094 weist ebenfalls auf die Schwierigkeiten der Cholinchloridherstellung hin und findet auch in GB-A Nr. 1 060 256 keine befriedigende Lösung im Hinblick auf Betriebssicherheit, Umsetzung unter hohem Druck, absatzweise Reaktion, Umsatz (Spalte 1, Zeile 42 bis Spalte 2, Zeile 12) und insbesondere die Abhängigkeit des Umsatz nimmt es vom pH, denn oberhalb von 90% Umsatz der pH-Wert rasch zu und erreicht pH-Werte um 12. Sie gibt an, dass solche pH-Werte Nebenreaktionen, die zu Verfärbungen führen, begünstigen und so auch das Verfahren nach GB-A Nr. 1 060 256 in kontinuierlichem Betrieb nur bei absatzweiser Umsetzung und Verzicht auf vollständigen Umsatz einen farblosen Endstoff liefert. In DE-B Nr. 2 115 094 wird diese Schwierigkeit des Verfahrens nach GB-A Nr. 1 060 256, welche zur Bildung von Braunfärbung führt, dadurch umgangen, dass man für eine gute Rückvermischung bzw. Bespülung der Reaktorwände mit Reaktionsprodukt sorgt.

Auch diese 2- bis 3-Stufenverfahren sind bezüglich der Vermeidung von Nebenprodukten im technischen Cholinchloridprozess, z. B. Ethylenchlorhydrin, β-β'-Dichlordiether, Ethylenglykol bzw. Polyglykolen oder freiem Triethylamin nicht befriedigend. Denn im Hinblick auf die spätere Verwendung (tierische Futtermittel) werden heute eine stabile farblose Cholinchloridlösung von grosser Lagerstabilität und eine hohe Reinheit des Cholinchlorids, vor allem von toxischen Nebenprodukten, z. B. Ethylenchlorhydrin und chlorhaltigen Ethern, verlangt. Auch muss bedacht werden, dass solche Nebenprodukte bei der Verwendung als tierisches Futtermittel möglicherweise auf Umwegen wiederum in die menschliche Nahrung gelangen können.

In DE-B Nr. 2 115 094 wird im Beispiel und in Spalte 3, Zeile 15 bis Spalte 4, Zeile 38, in einer bevorzugten Ausführungsform ein Zweistufenverfahren zur Herstellung von Cholinchlorid aus Trimethylammoniumchlorid und Ethylenoxid beschrieben und darauf hingewiesen, dass mit einem Überschuss von Ethylenoxid (2 bis 18 Mol-%) bei der Umsetzung mit Triethylammoniumchlorid bei pH 12 und 65 °C gearbeitet wird. Der Überschuss an nicht umgesetztem Ethylenoxid begünstigt Nebenreaktionen, die z. B. 0,5 Gew.-% des hochtoxischen Ethylenchlorhydrins in der Reaktionslösung bilden (Spalte 3, Zeilen 47 bis 54). Eine spätere Entfernung des Ethylenchlorhydrins aus der technischen Cholinchloridlösung ist kaum möglich.

Es wurde nun gefunden, dass man Salze des Cholins durch Umsetzung von Trimethylamin, Ethylenoxid und einer Säure kontinuierlich vorteilhaft herstellt, wenn man Trimethylamin, Ethylenoxid und eine Säure kontinuierlich miteinander vermischt und in Gegenwart von Wasser bei pH-Werten oberhalb 9 bis 12 und einer Temperatur von 50 bis 80 °C umsetzt.

Die Umsetzung kann für den Fall der Verwendung von Salzsäure durch das folgende Formelschema wiedergegeben werden:

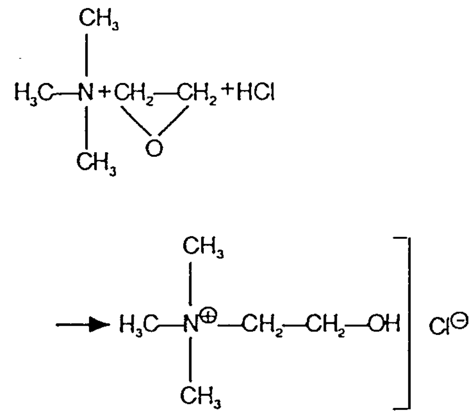

Im Hinblick auf die bekannten Verfahren liefert das Verfahren nach der Erfindung auf einfacherem und wirtschaftlicherem Wege Cholinsalze in teilweise besserer Ausbeute und Reinheit. Eine Umsetzung in Stufen ist nicht notwendig. Das nach dem erfindungsgemässen Verfahren erhaltene Cholinsalz ist praktisch frei von Chlorhydrin und anderen Nebenprodukten und in der Regel ohne weitere Reinigung ersetzbar. Alle diese vorteilhaften Ergebnisse sind im Hinblick auf den Stand der Technik überraschend. Im Hinblick auf GB-A Nr. 1 060 256 und DE-B Nr. 2 115 094 konnte nicht erwartet werden, dass in dem einbadigen, einstufigen, erfindungsgemässen Verfahren und anstelle des Salzes mit freiem Trimethylamin als Ausgangsstoff kontinuierlich ein farbloses Cholinchlorid in besserer Ausbeute an reinem Endstoff hergestellt wird. Es war mit Bezug auf diesen Stand der Technik überraschend, dass die Umsetzung in einem üblichen Rührreaktor ohne eine komplizierte Zerstäubungs- und Bespülungsvorrichtung durchgeführt werden kann. Auch war nicht zu erwarten, dass die vorteilhaften erfindungsgemässen Ergebnisse ohne die in GB-A Nr. 1 060 256 beanspruchte Betriebsweise unter hohem Druck und ohne Nachreaktionsstufe erhalten werden. Das erfindungsgemäss hergestellte Cholinchlorid ist überraschend praktisch frei von toxischen Nebenprodukten und muss keiner weiteren Reinigung unterzogen werden (S. 3, Zeilen 20 bis 28). Gerade angesichts der erfindungsgemässen gleichzeitigen Reaktion von drei Ausgangsstoffen hätte man einen grösseren Anteil an Nebenprodukten erwartet.

Man kann die 3 Ausgangsstoffe im Überschuss jedes Ausgangsstoffes zum anderen oder vorteilhaft in einer Menge von 1 bis 1,10, bevorzugt 1 mol Ethylenoxid oder 0,95 bis 1, bevorzugt 1 Eq Säure je Mol Trimethylamin, umsetzen. Wasser wird zweckmässig in einer Menge von 10 bis 50, insbesondere von 20 bis 40 Gew.-% bezogen auf die Gewichtsmenge Säure, verwendet. Es ist dabei gleichgültig, ob man das Wasser als solches dem Ausgangsgemisch zuführt oder zusammen mit Säure bzw. Trimethylamin. Trimethylamin wird entweder gasförmig oder als wässerige, zweckmässig 40- bis 45-gew.-%ige Lösung verwendet. Ethylenoxid wird vorteilhaft gasförmig zur Reaktion gebracht.

Als Säure wird Salzsäure und Chlorwasserstoff verwendet.

Die Umsetzung wird bei einer Temperatur von 50 bis 80, zweckmässig 65 bis 80, insbesondere 65 bis 76°C, drucklos oder unter Druck durchgeführt. Der pH-Wert wird mit Hilfe einer Glaselektrode gemessen. Die Umsetzung der 3 Reaktanten Trimethylamin, Ethylenoxid und Salzsäure erfolgt bei pH-Werten oberhalb 9 bis 12, zweckmässig 9,1 bis 12, vorteilhaft 9,5 bis 11,5, vorzugsweise 10 bis 11,5.

Die Reaktion kann wie folgt durchgeführt werden: Gleichzeitig werden kontinuierlich Trimethylamin, Ethylenoxid und Säure zusammen mit Wasser in einen Kontinuereaktor, z.B. Rohrreaktor, eingegeben. Bei einer bevorzugten Ausführungsform werden unter Rühren der Chlorwasserstoff als konzentrierte Salzsäure und Trimethylamin und Ethylenoxid gasförmig in den Reaktor so kontinuierlich eingeführt, dass die Eingabe unter der Oberfläche des Ausgangsgemischs bzw. Reaktionsgemischs erfolgt. Die Verweilzeit des Reaktionsgemischs beträgt im Reaktor 60 bis 300 min.

Das nach dem Verfahren der Herstellung herstellbare Cholinchlorid ist ein wertvoller Ausgangsstoff für Pharmazeutika und Futtermittel. Bezüglich der Verwendung verweisen wir auf vorgenannte Druckschriften.

Die in den Beispielen genannten Teile sind Gewichtsteile.

Beispiel 1

In einen Reaktor werden stündlich 123,7 Teile 29,5-gew.-%ige HCl, 61,95 Teile Trimethylamin und 48,4 Teile Ethylenoxid aus getrennten Behältern gleichzeitig und kontinuierlich unter die Oberfläche des Ausgangsgemischs eingebracht. Die Temperatur wird bei 70 bis 75°C gehalten. Der pH-Wert wird im Bereich von 10 bis 10,5 gehalten. Die Verweilzeit im Reaktor beträgt 240 min. Das Reaktionsgut wird zur Entfernung von Spuren Trimethylamin bzw. Ethylenoxid in einer Rieselkolonne im Gegenstrom mit Stickstoff ausgeblasen. Man erhält stündlich 225 Teile einer wasserhellen 60-gew.-%igen Cholinchloridlösung (96% der Theorie, bezogen auf Trimethylamin) mit einem Cholinchlorid vom Fp. 247°C (Zers.).

## Patentanspruch

Verfahren zur kontinuierlichen Herstellung von Cholinchlorid durch Umsetzung von Trimethylamin, Ethylenoxid und Salzsäure, dadurch gekennzeichnet, dass man Trimethylamin, Ethylenoxid und Salzsäure bzw. Chlorwasserstoff kontinuierlich miteinander vermischt und in Gegenwart von Wasser bei pH-Werten oberhalb 9 bis 12 und einer Temperatur von 50 bis 80°C umsetzt.

## Claim

A process for the continuous production of choline chloride by reaction of trimethylamine, ethylene oxide and hydrochloric acid, wherein trimethylamine, ethylene oxide and hydrochloric acid or hydrogen chloride are continuously mixed with one another and reacted in the presence of water at a pH from above 9 to 12 and at from 50 to 80°C.

## Revendication

Procédé de préparation en continu de chlorure de choline par réaction de triméthylamine, d'oxyde d'éthylène et d'acide chlorhydrique, caractérisé en ce qu'on mélange entre eux la triméthylamine, l'oxyde d'éthylène et de l'acide chlorhydrique ou du gaz chlorhydrique de façon continue et on les fait réagir en présence d'eau à un pH compris entre une valeur supérieure à 8 et 12 et à une température de 50 à 80°C.